# EUROPEAN PATENT SPECIFICATION

(11) **EP 4 309 714 B1**
(45) Date of publication and mention of the grant of the patent: **16.07.2025**
(21) Application number: 23729930.0
(22) Date of filing: 23.03.2023
(51) Int. Cl.: A61M 15/00, A61M 11/02, A61M 15/06

(54) **INHALER**
INHALATOR
INHALATEUR

(30) Priority: 30.05.2022 KR 20220065845
(43) Date of publication of application: 24.01.2024
(73) Proprietor: KT & G Corporation, Daejeon 34337 (KR)
(72) Inventor: KIM, Tae Heon, Daejeon 34128 (KR); KIM, Jae Hyun, Daejeon 34128 (KR); LEE, Mi Jeong, Daejeon 34128 (KR); JEONG, Minseok, Daejeon 34128 (KR); JUNG, Yongmi, Daejeon 34128 (KR); JEOUNG, Eun Mi, Daejeon 34128 (KR); CHUNG, Tae Young, Daejeon 34128 (KR); HAN, Seung Kyu, Daejeon 34128 (KR)
(74) Representative: Ter Meer Steinmeister & Partner
(86) International application number: PCT/KR2023/003825
(87) International publication number: WO 2023/234528

(56) References cited:
- EP-A1- 4 302 809
- WO-A1-2011/147687
- WO-A1-2020/007413
- WO-A1-99/49916
- WO-A2-2012/045683
- CN-A- 113 679 910
- JP-A- 2006 505 374
- KR-A- 20050 004 845
- KR-A- 20120 037 938

## Description

### Technical Field

An inhaler is disclosed.

### Background Art

In general, an inhaler is a device used to inhale a composition such as a medicine through the oral cavity or nasal cavity as a liquid or gas in the process of inhalation. Such an inhaler includes a container for accommodating an inhalable composition, and the composition is sprayed from the container through a thin tube to the oral cavity or nasal cavity through an intake to be finally inhaled by a user.

The above description has been possessed or acquired by the inventor(s) in the course of conceiving the present disclosure and is not necessarily an art publicly known before the present application is filed.

Prior Document: Korean Patent Gazette No. 10-2021229
EP 4 302 809 A1 is prior art according to Art. 54(3) and relates to an inhaler as shown in Fig. 1. WO 2020/007413 A1 relates to an aerosol dispersion device comprising a pressurized aerosol can in which a consumable substance may be stored, wherein the aerosol can comprises a valve which can be actuated by a lever for releasing the consumable substance.

### Disclosure of the Invention

### Technical Goals

An object according to an embodiment is to provide an inhaler including a filling lever that facilitates filling of a reservoir with an inhalable composition accommodated in a canister.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

According to the claimed invention for achieving the above object, an inhaler includes a housing having one surface, the other surface opposite to the one surface, and a plurality of side surfaces connecting the one surface and the other surface, a canister which is mounted interchangeably inside the housing and accommodates an inhalable composition, a reservoir which is positioned inside the housing and to which one side of the canister is connected, and a lever having one side forming a portion of the housing, and the other side being movable between a bottom surface of the canister and the other surface of the housing. When one side of the lever is pressed, the other side of the lever moves the canister in a direction toward the reservoir and some of the inhalable composition in the canister are stored in the reservoir.

According to an aspect, when the lever is pressed, the lever may move in a first direction from one side of the housing toward the canister.

According to an aspect, the reservoir may be disposed adjacent to the one surface of the housing, the other side of the canister may extend from the one side of the canister to the other surface of the housing, and when the lever is pressed, the canister may move in a second direction from the other surface to the one surface of the housing.

According to the claimed invention, the lever includes a first lever member which forms a portion of one side surface of the plurality of side surfaces of the housing, and a second lever member which extends from the first lever member toward the canister, and when the first lever member is pressed, the second lever member moves in a first direction from one side of the housing toward the canister to be inserted between the bottom surface of the canister and the other surface of the housing, and the canister moves in a second direction from the other surface to the one surface of the housing.

According to an aspect, the second lever member may include an inclined surface formed in a shape in which a height decreases along the first direction, and when the first lever member is pressed, the inclined surface may move the bottom surface of the canister in the second direction.

According to an aspect, the lever may further include a third lever member having one end fixed to the inside of the housing, and the other end coupled to the first lever member, and when the first lever member is pressed, the third lever member may be compressed in the first direction, and when the first lever member is not pressed, the third lever member may be restored to return the second lever member to its original position.

According to an aspect, the canister may include an accommodation portion which accommodates the inhalable composition, and an injection hole having one side connected to the reservoir and the other side positioned in the accommodation portion, and the injection hole may be operated to be opened or closed with respect to the reservoir.

According to an aspect, when the lever is pressed, the injection hole may be opened to allow the inhalable composition to move to the reservoir, and when the lever is not pressed, the injection hole may be closed.

According to an aspect, the inhaler may further an elastic member which is provided in the injection hole, and the elastic member may be compressed when the canister moves in a second direction from the other surface to the one surface of the housing, and return the canister to its original position when no external force is applied to the canister.

According to an aspect, when the lever is pressed, the reservoir may be filled with the inhalable composition in an intake volume for 10 to 15 times of puffs.

### Effects

According to the inhaler according to an embodiment, it is easy to fill the reservoir with the inhalable composition accommodated in the canister by pressing the filling lever.

The effects of the inhaler are not limited to the above-mentioned effects, and other unmentioned effects can be clearly understood from the above description by those having ordinary skill in the technical field to which the present disclosure pertains.

### Brief Description of Drawings

FIG. 1 is a perspective view of an inhaler according to an embodiment.
FIG. 2 is a cross-sectional view of an inhaler according to an embodiment.
FIG. 3 is a cross-sectional view of an inhaler in a state where a lever is not pressed according to an embodiment
FIG. 4 is a cross-sectional view of an inhaler in a state where a lever is pressed according to an embodiment

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions of the embodiments may be applicable to the following embodiments and thus, duplicated descriptions will be omitted for conciseness.

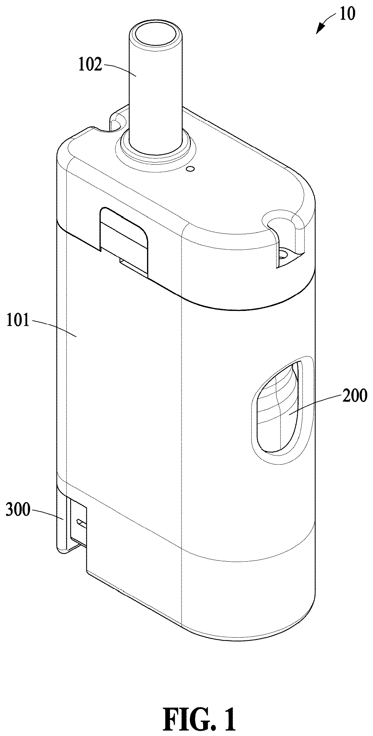

FIG. 1 is a perspective view of an inhaler 10 according to an embodiment.

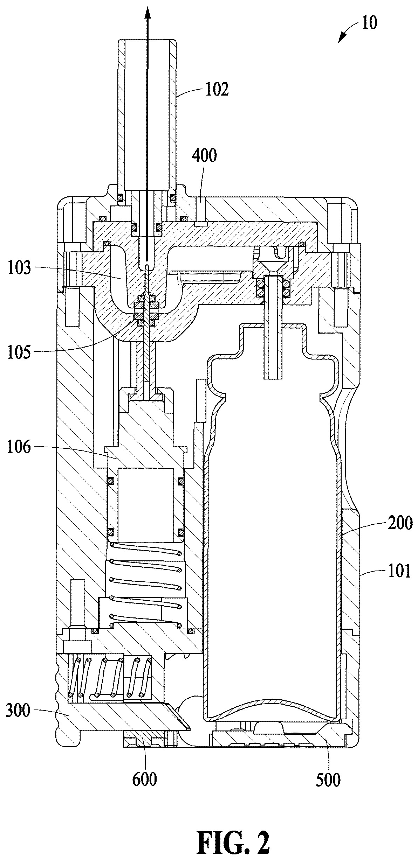

FIG. 2 is a cross-sectional view of the inhaler 10 according to an embodiment.

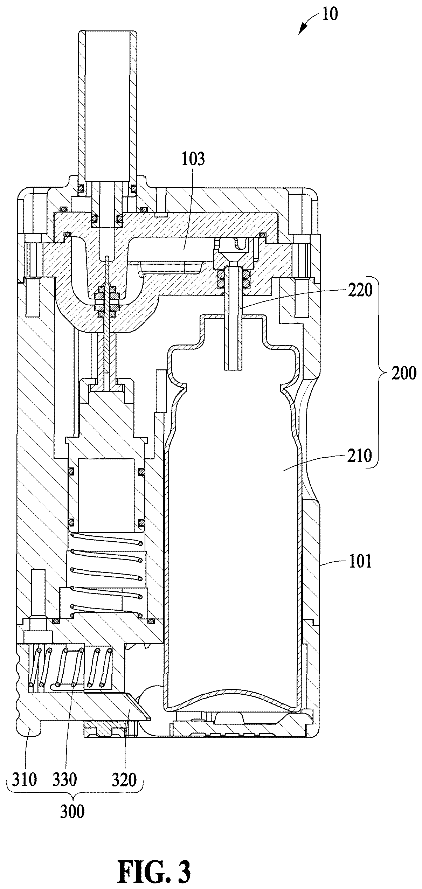

FIG. 3 is a cross-sectional view of the inhaler 10 in a state where a lever 300 is not pressed according to an embodiment.

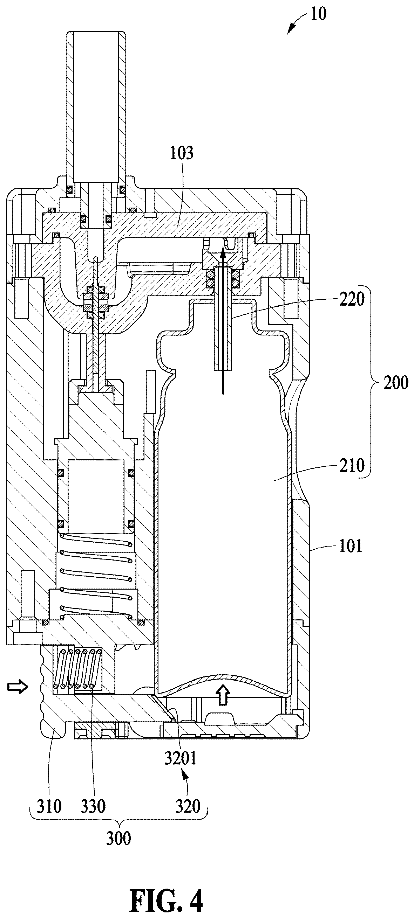

FIG. 4 is a cross-sectional view of the inhaler 10 in a state where the lever 300 is pressed according to an embodiment.

Referring to FIG. 1, the inhaler 10 according to an embodiment may include a housing 101, a mouthpiece 102, a reservoir 103, a canister 200, and the lever 300.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIGS. 1 and 2, the reservoir 103 may be disposed inside the housing 101. Since one side of the canister 200 is connected to the reservoir 103, the inhalable composition accommodated in the canister 200 may move to be stored in the reservoir 103. In addition, as a user applies a suction force through the mouthpiece 102, the inhalable composition stored in the reservoir 103 may be discharged through the mouthpiece 102 in the form of an aerosol, and thus, the inhalable composition stored in the reservoir 103 may be gradually consumed.

The canister 200 may be mounted inside the housing 101. In this case, the canister 200 may be mounted interchangeably inside the housing 101. In addition, the canister 200 may accommodate an inhalable composition therein. A certain amount of the composition of the canister 200 may be filled into the reservoir 103.

The lever 300 may be pressed by a user to fill the composition accommodate in the canister 200 into the reservoir 103. The lever 300 may form one side surface of the housing 101 and may be positioned adjacent to the second surface. When a user presses the lever 300, the lever 300 may push a bottom surface of the canister 200 up so that an injection hole 220 of the canister 200 communicates with the reservoir 103, and the composition may move from the canister 200 to the reservoir 103 through the injection hole 220.

Hereinafter, a direction from one side surface of the housing 101, where the lever 300 is disposed, to the canister 200 is defined as a first direction, and a direction from the second surface to the first surface of the housing 101 is defined as a second direction.

Referring to FIG. 2, in the inhaler 10 according to an embodiment, a user may visually identify a remaining amount of the composition stored in the reservoir 103 through a viewing window (not shown) provided in the housing 101. In addition, the inhaler 10 according to an embodiment may include a counter (not shown) that counts the number of times of filling in connection with a vertical movement of the canister 200 during the filling of the reservoir 103, and a display window (not shown) that displays a remaining amount of a composition in the canister 200. The composition stored in the reservoir 103 may be sprayed in the form of an aerosol, as a user applies a suction force to the mouthpiece 102. At this time, an inhale interlocking valve 105 that opens and closes the reservoir 103 may be operated by the suction force, and the opening and closing operation of the inhale interlocking valve 105 may be controlled by a piston 106. In addition, the reservoir 103 may be provided with a relief valve (not shown), and a relief vent hole 400 may be provided on the first surface of the housing 101. This relief valve is interlocked with the lever 300 through a relief bar (not shown), a relief bar moving projection (not shown), and the like. Therefore, the relief valve may be opened first to discharge a residual gas in the reservoir 103, immediately before the lever 300 pushes the canister 200 up to fill the reservoir 103. Additionally, the inhaler 10 according to an embodiment may include a cover 500 and a locking device 600 for preventing the detachment of the canister 200.

The lever 300 that facilitates the filling of a reservoir with an inhalable composition of a canister is applied to the inhaler 10 described above, and the specific configuration and operating mechanism of the lever 300 will be described below in detail with reference to FIGS. 3 and 4.

Referring to FIGS. 3 and 4, one side of the lever 300 of the inhaler 10 according to an embodiment may form a portion of the housing 101. Also, the other side thereof is movable between the bottom surface of the canister 200 and the second surface of the housing 101. When the one side of the lever 300 is pressed by a user, the other side of the lever 300 may move the canister 200 in a direction to the reservoir 103. Accordingly, some of the inhalable composition in the canister 200 may be moved to and stored in the reservoir 103.

For example, the lever 300 may move in the first direction when pressed by a user. At this time, the canister 200 may move in the second direction by the lever 300.

For example, the reservoir 103 may be disposed adjacent to the first surface of the housing 101. The canister 200 may be disposed between the reservoir 103 and the second surface of the housing 101. That is, the canister 200 may be vertically disposed on the second surface of the housing 101, and at this time, the canister 200 may be disposed adjacent to a side surface opposite to the one side surface of the housing 101 formed by the lever 300. That is, the canister 200 may be disposed so that the bottom surface thereof is adjacent to the second surface of the housing 101 and an upper portion of the canister 200 may be adjacent to the first surface of the housing 101.

Referring to FIG. 3, the lever 300 may include a first lever member 310 and a second lever member 320.

The first lever member 310 may form a portion of one side surface of the plurality of side surfaces of the housing 101.

The second lever member 320 may extend from the first lever member 310 toward the canister 200.

As shown in FIG. 4, when the first lever member 310 is pressed by a user and moves in the first direction, the second lever member 320 may move in the first direction together to be inserted between the bottom surface of the canister 200 and the second surface of the housing 101. Accordingly, the canister 200 may move in the second direction. That is, the second lever member 320 may push the canister 200 up toward the reservoir 103.

Referring to FIG. 4, the second lever member 320 may include an inclined surface 3201. The inclined surface 3201 may be formed in a shape in which a height decreases along the first direction. That is, a height of a portion close to the canister 200 may be smaller than a height of a portion close to the first lever member 310. When the first lever member 310 is pressed, the inclined surface 3201 may be inserted between the bottom surface of the canister 200 and the second surface of the housing 101. At this time, the inclined surface 3201 may come into contact with the bottom surface of the canister 200, and a corner portion of the bottom surface may slide on the inclined surface 3201. Accordingly, the bottom surface of the canister 200 may be pushed up in the second direction. That is, when the first lever member 310 is pressed, the canister 200 may move in the second direction due to the shape of the inclined surface 3201.

Referring again to FIGS. 3 and 4, the lever 300 of the inhaler 10 according to an embodiment may further include a third lever member 330.

One end of the third lever member 330 may be fixed inside the housing, and the other end thereof may be coupled to the first lever member 310. In addition, the third lever member 330 may be positioned between the first lever member 310 and the second lever member 320. The third lever member 330 may be provided with, for example, a compression spring. As shown in FIG. 4, the third lever member 330 may be compressed in the first direction when the first lever member 310 is pressed. Also, when the first lever member 310 is not pressed, the third lever member 330 may be restored to return the second lever member 320 to its original position as shown in FIG. 3. As the second lever member 320 returns to its original position, the canister 200 may also return to its original position and the filling of the reservoir 103 with the inhalable composition may be stopped.

Specifically, the canister 200 of the inhaler 10 according to an embodiment may include an accommodation portion 210 and the injection hole 220.

The accommodation portion 210 may accommodate an inhalable composition.

One side of the injection hole 220 may be connected to the reservoir 103 and the other side thereof may be positioned in the accommodation portion 210. The injection hole 220 may be opened or closed with respect to the reservoir 103. For example, the injection hole 220 may be opened when the first lever member 310 is pressed. When the injection hole 220 is opened, the inhalable composition accommodated in the accommodation portion 210 may be allowed to move to the reservoir 103. On the other hand, when the first lever member 310 is not pressed, the injection hole 220 may be closed. When the injection hole 220 is closed, a moving path of the inhalable composition to the reservoir 103 may be blocked, and accordingly, the filling of the reservoir 103 may be stopped.

Meanwhile, when the lever 300 is pressed in a state in which the inhalable composition in the reservoir 103 runs out, the reservoir 103 may be filled with the inhalable composition in an intake volume for 10 to 15 times of puffs. Also, in order to fill the reservoir 103 with the inhalable composition in an intake volume for 10 to 15 times of puffs, the lever 300 may need to be pressed for 3 to 5 seconds.

The operation of opening and closing the injection hole 220 may be controlled according to whether a user presses the lever 300.

As described above, when the first lever member 310 is pressed, the second lever member 320 may push up the bottom surface of the canister 200, that is, a bottom surface of the accommodation portion 210. At this time, as shown in FIG. 4, the accommodation portion 210 moves in the second direction, but one side of the injection hole 220 may be fixed to the reservoir 103. That is, the position of the injection hole 220 in the housing 101 does not change, but the position of the other side of the injection hole 220 in the accommodation portion 210 may change due to the rising of the accommodation portion 210 in the second direction.

For example, when the other side of the injection hole 220 is pressed deeply into the accommodation portion 210, the injection hole may be opened. Accordingly, the inhalable composition in the accommodation portion 210 may move to the reservoir 103 through the injection hole 220. At this time, the inhalable composition may move by a pressure difference between the reservoir 103 and the accommodation portion 210. That is, when the inhalable composition filled in the reservoir 103 by a user is consumed by aerosol discharge, the pressure in the reservoir 103 may be reduced, and therefore, when the injection hole 220 is opened, the inhalable composition may move from the accommodation portion 210 to the reservoir 103. On the other hand, when the reservoir 103 is sufficiently filled with the inhalable composition, the inhalable composition may not additionally move to the reservoir 103 even if a user presses the lever 300 to open the injection hole 220.

In addition, the canister 200 may further include an elastic member (not shown) that is positioned in the accommodation portion 210 and compressed and restored according to a pressure applied to the injection hole 220. The elastic member may be provided with, for example, a compression spring.

As described above, since one side of the injection hole 220 is fixed to the reservoir 103, the one side of the injection hole 220 may be pressed when the accommodation portion 210 receives an external force in the second direction by the lever 300. At this time, the elastic member is compressed when the one side of the injection hole 220 is pressed, and thus, the accommodation portion 210 may move in the second direction with the injection hole 220 as an axis, as shown in FIG. 4. In addition, when the lever 300 is not pressed by a user and returns to its original position, the external force of the lever 300 relative to the canister 200 is also removed. At this time, the accommodation portion 210 may move in a direction opposite to the second direction as the elastic member is restored. That is, if no external force is applied to the bottom surface of the accommodation portion 210 by the second lever member 320, the elastic member may return the accommodation portion 210 to its original position as shown in FIG. 3.

In the inhaler 10 according to an embodiment, as a user presses the lever 300, the reservoir 103 may be easily filled with an inhalable composition accommodated in the canister 200.

## Claims

1. An inhaler (10) comprising:
a housing (101) having one surface, the other surface opposite to the one surface, and a plurality of side surfaces connecting the one surface and the other surface;
a canister (200) which is mounted interchangeably inside the housing (101) and accommodates an inhalable composition;
a reservoir (103) which is positioned inside the housing (101) and to which one side of the canister (200) is connected; and
a lever (300) having one side forming a portion of the housing (101), and the other side being movable between a bottom surface of the canister (200) and the other surface of the housing (101),
wherein, when one side of the lever (300) is pressed, the other side of the lever (300) moves the canister (200) in a direction toward the reservoir (103) and some of the inhalable composition in the canister (200) are stored in the reservoir (103),
wherein the lever (300) further comprises:
a first lever member (310) which forms a portion of one side surface of the plurality of side surfaces of the housing (101); and
a second lever member (320) which extends from the first lever member (310) toward the canister (200), and
wherein, when the first lever member (310) is pressed, the second lever member (320) moves in a first direction from one side of the housing (101) toward the canister (200) to be inserted between the bottom surface of the canister (200) and the other surface of the housing (101), and the canister (200) moves in a second direction from the other surface to the one surface of the housing (101).

2. The inhaler (10) of claim 1, wherein, when the lever (300) is pressed, the lever (300) moves in a first direction from one side of the housing (101) toward the canister (200).

3. The inhaler (10) of claim 1,
wherein the reservoir (103) is disposed adjacent to the one surface of the housing (101),
wherein the other side of the canister (200) extends from the one side of the canister (200) to the other surface of the housing (101), and
wherein, when the lever (300) is pressed, the canister (200) moves in a second direction from the other surface to the one surface of the housing (101).

4. The inhaler (10) of claim 1,
wherein the second lever member (320) comprises an inclined surface (3201) formed in a shape in which a height decreases along the first direction, and
wherein, when the first lever member (310) is pressed, the inclined surface (3201) moves the bottom surface of the canister (200) in the second direction.

5. The inhaler (10) of claim 1,
wherein the lever (300) further comprises a third lever member (330) having one end fixed to the inside of the housing (101), and the other end coupled to the first lever member (310), and
wherein, when the first lever member (310) is pressed, the third lever member (330) is compressed in the first direction, and when the first lever member (310) is not pressed, the third lever member (330) is restored to return the second lever member (320) to its original position.

6. The inhaler (10) of claim 1, wherein the canister (200) comprises:
an accommodation portion (210) which accommodates the inhalable composition; and
an injection hole (220) having one side connected to the reservoir (103) and the other side positioned in the accommodation portion (210), and
wherein the injection hole (220) is operated to be opened or closed with respect to the reservoir (103).

7. The inhaler (10) of claim 6, wherein, when the lever (300) is pressed, the injection hole (220) is opened to allow the inhalable composition to move to the reservoir (103), and when the lever (300) is not pressed, the injection hole (220) is closed.

8. The inhaler (10) of claim 6, further comprising: an elastic member which is provided in the injection hole (220),
wherein the elastic member is compressed when the canister (200) moves in a second direction from the other surface to the one surface of the housing (101), and returns the canister (200) to its original position when no external force is applied to the canister (200).

9. The inhaler (10) of claim 1, wherein, when the lever (300) is pressed, the reservoir (103) is filled with the inhalable composition in an intake volume for 10 to 15 times of puffs.

## Patentansprüche

1. Inhalator (10), der umfasst:
ein Gehäuse (101) mit einer Fläche, einer anderen Fläche gegenüber der einen Fläche und mehreren Seitenflächen, die die eine Fläche und die andere Fläche verbinden;
einen Behälter (200), der austauschbar innerhalb des Gehäuses (101) angebracht ist und eine inhalierbare Zusammensetzung aufnimmt;
ein Reservoir (103), das innerhalb des Gehäuses (101) positioniert ist und mit der einen Seite des Behälters (200) verbunden ist; und
einen Hebel (300) mit einer Seite, die einen Teil des Gehäuses (101) bildet, und der anderen Seite, die zwischen einer unteren Fläche des Behälters (200) und der anderen Fläche des Gehäuses (101) beweglich ist,
wobei dann, wenn eine Seite des Hebels (300) gedrückt wird, die andere Seite des Hebels (300) den Behälter (200) in einer Richtung zu dem Reservoir (103) bewegt und einiges der inhalierbaren Zusammensetzung in dem Behälter (200) in dem Reservoir (103) gespeichert wird,
wobei der Hebel (300) ferner umfasst:
ein erstes Hebelelement (310), das einen Teil einer Seitenfläche der mehreren Seitenflächen des Gehäuses (101) bildet; und
ein zweites Hebelelement (320), das sich von dem ersten Hebelelement (310) zu dem Behälter (200) erstreckt,
wobei dann, wenn das erste Hebelelement (310) gedrückt wird, sich das zweite Hebelelement (320) in einer ersten Richtung von einer Seite des Gehäuses (101) zu dem Behälter (200) bewegt, um zwischen der unteren Fläche des Behälters (200) und der anderen Fläche des Gehäuses (101) eingeführt zu werden, und sich der Behälter (200) in einer zweiten Richtung von der anderen Fläche zu der einen Fläche des Gehäuses (101) bewegt.

2. Inhalator (10) nach Anspruch 1, wobei dann, wenn der Hebel (300) gedrückt wird, sich der Hebel (300) in einer ersten Richtung von einer Seite des Gehäuses (101) zu dem Behälter (200) bewegt.

3. Inhalator (10) nach Anspruch 1,
wobei das Reservoir (103) angrenzend an die eine Fläche des Gehäuses (101) angeordnet ist,
wobei sich die andere Seite des Behälters (200) von der einen Seite des Behälters (200) zu der anderen Fläche des Gehäuses (101) erstreckt und
wobei dann, wenn der Hebel (300) gedrückt wird, sich der Behälter (200) in einer zweiten Richtung von der anderen Fläche zu der einen Fläche des Gehäuses (101) bewegt.

4. Inhalator (10) nach Anspruch 1,
wobei das zweite Hebelelement (320) eine geneigte Fläche (3201) aufweist, die in einer Form gebildet ist, in der eine Höhe entlang der ersten Richtung abnimmt, und
wobei dann, wenn das erste Hebelelement (310) gedrückt wird, die geneigte Fläche (3201) die untere Fläche des Behälters (200) in der zweiten Richtung bewegt.

5. Inhalator (10) nach Anspruch 1,
wobei der Hebel (300) ferner ein drittes Hebelelement (330) mit einem Ende, das im Inneren des Gehäuses (101) befestigt ist, und einem anderen Ende, das an das erste Hebelelement (310) gekoppelt ist, umfasst und
wobei dann, wenn das erste Hebelelement (310) gedrückt wird, das dritte Hebelelement (330) in der ersten Richtung komprimiert wird und dann, wenn das erste Hebelelement (310) nicht gedrückt wird, das dritte Hebelelement (330) wiederhergestellt wird, um das zweite Hebelelement (320) in seine Ausgangsposition zurückzuführen.

6. Inhalator (10) nach Anspruch 1, wobei der Behälter (200) umfasst:
einen Aufnahmeteil (210), der die inhalierbare Zusammensetzung aufnimmt; und
eine Injektionsloch (220) mit einer Seite, die mit dem Reservoir (103) verbunden ist, und der anderen Seite, die in dem Aufnahmeteil (210) positioniert ist,
wobei das Injektionsloch (220) bedient wird, um in Bezug auf das Reservoir (103) geöffnet oder geschlossen zu sein.

7. Inhalator (10) nach Anspruch 6, wobei dann, wenn der Hebel (300) gedrückt wird, das Injektionsloch (220) geöffnet wird, um der inhalierbaren Zusammensetzung zu erlauben, sich zu dem Reservoir (103) zu bewegen, und dann, wenn der Hebel (300) nicht gedrückt wird, das Injektionsloch (220) geschlossen ist.

8. Inhalator (10) nach Anspruch 6, der ferner umfasst: ein elastisches Element, das in dem Injektionsloch (220) vorgesehen ist,
wobei das elastische Element komprimiert wird, wenn sich der Behälter (200) in einer zweiten Richtung von der anderen Fläche zu der einen Fläche des Gehäuses (101) bewegt, und den Behälter (200) in seine Ausgangsposition zurückführt, wenn keine äußere Kraft auf den Behälter (200) ausgeübt wird.

9. Inhalator (10) nach Anspruch 1, wobei dann, wenn der Hebel (300) gedrückt wird, das Reservoir (103) mit der inhalierbaren Zusammensetzung in ein Aufnahmevolumen für 10 bis 15 Züge gefüllt wird.

## Revendications

1. Inhalateur (10) comportant :
un boîtier (101) ayant une première surface, une seconde surface opposée à la première surface, et une pluralité de surfaces latérales reliant la première surface et la seconde surface ;
une cartouche (200) qui est montée de manière interchangeable à l'intérieur du boîtier (101) et reçoit une composition inhalable ;
un réservoir (103) qui est positionné à l'intérieur du boîtier (101) et auquel un premier côté de la cartouche (200) est relié ; et
un levier (300) ayant un premier côté formant une partie du boîtier (101) et un second côté étant mobile entre une surface de fond de la cartouche (200) et la seconde surface du boîtier (101),
dans lequel, lorsqu'un premier côté du levier (300) est pressé, le second côté du levier (300) déplace la cartouche (200) dans une direction allant vers le réservoir (103) et une partie de la composition inhalable dans la cartouche (200) est stockée dans le réservoir (103),
dans lequel le levier (300) comporte en outre :
un premier élément de levier (310) qui forme une partie d'une surface latérale de la pluralité de surfaces latérales du boîtier (101) ; et
un deuxième élément de levier (320) qui s'étend à partir du premier élément de levier (310) vers la cartouche (200), et
dans lequel, lorsque le premier élément de levier (310) est pressé, le deuxième élément de levier (320) se déplace dans une première direction allant d'un premier côté du boîtier (101) vers la cartouche (200) pour être inséré entre la surface de fond de la cartouche (200) et la seconde surface du boîtier (101), et la cartouche (200) se déplace dans une seconde direction allant de la seconde surface à la première surface du boîtier (101).

2. Inhalateur (10) selon la revendication 1, dans lequel, lorsque le levier (300) est pressé, le levier (300) se déplace dans une première direction allant d'un premier côté du boîtier (101) vers la cartouche (200).

3. Inhalateur (10) selon la revendication 1,
dans lequel le réservoir (103) est disposé au voisinage de la première surface du boîtier (101),
dans lequel le second côté de la cartouche (200) s'étend à partir du premier côté de la cartouche (200) jusqu'à la seconde surface du boîtier (101), et
dans lequel, lorsque le levier (300) est pressé, la cartouche (200) se déplace dans une seconde direction allant de la seconde surface à la première surface du boîtier (101).

4. Inhalateur (10) selon la revendication 1,
dans lequel le deuxième élément de levier (320) comporte une surface inclinée (3201) formée avec une forme dans laquelle une hauteur diminue le long de la première direction, et
dans lequel, lorsque le premier élément de levier (310) est pressé, la surface inclinée (3201) déplace la surface de fond de la cartouche (200) dans la seconde direction.

5. Inhalateur (10) selon la revendication 1,
dans lequel le levier (300) comporte en outre un troisième élément de levier (330) ayant une première extrémité fixée à l'intérieur du boîtier (101), et la seconde extrémité couplée au premier élément de levier (310), et
dans lequel, lorsque le premier élément de levier (310) est pressé, le troisième élément de levier (330) est comprimé dans la première direction, et lorsque le premier élément de levier (310) n'est pas pressé, le troisième élément de levier (330) est rappelé pour ramener le deuxième élément de levier (320) à sa position d'origine.

6. Inhalateur (10) selon la revendication 1, dans lequel la cartouche (200) comporte :
une partie de réception (210) qui reçoit la composition inhalable ; et
un trou d'injection (220) ayant un côté relié au réservoir (103) et l'autre côté positionné dans la partie de réception (210), et
dans lequel le trou d'injection (220) fonctionne pour être ouvert ou fermé par rapport au réservoir (103).

7. Inhalateur (10) selon la revendication 6, dans lequel, lorsque le levier (300) est pressé, le trou d'injection (220) est ouvert pour permettre à la composition inhalable de se déplacer jusqu'au réservoir (103), et lorsque le levier (300) n'est pas pressé, le trou d'injection (220) est fermé.

8. Inhalateur (10) selon la revendication 6, comportant en outre : un élément élastique qui est prévu dans le trou d'injection (220),
dans lequel l'élément élastique est comprimé lorsque la cartouche (200) se déplace dans une seconde direction allant de la seconde surface à la première surface du boîtier (101), et ramène la cartouche (200) à sa position d'origine lorsqu'aucune force externe n'est appliquée à la cartouche (200).

9. Inhalateur (10) selon la revendication 1, dans lequel, lorsque le levier (300) est pressé, le réservoir (103) est rempli de la composition inhalable avec un volume d'aspiration correspondant à 10 à 15 bouffées.
